# EUROPÄISCHE PATENTSCHRIFT

(11) **EP 0 508 215 B1**
(45) Veröffentlichungstag und Bekanntmachung des Hinweises auf die Patenterteilung: **15.05.1996**
(21) Anmeldenummer: 92105101.7
(22) Anmeldetag: 25.03.1992
(51) Int. Cl.: C07D 213/61

(54) **Verfahren zur Herstellung von 2-Chlorpyridinen**
Process for the preparation of 2-chloro-pyridines
Procédé pour la préparation de chloro-2-pyridines

(30) Priorität: 06.04.1991 DE 4111215
(43) Veröffentlichungstag der Anmeldung: 14.10.1992
(73) Patentinhaber: BAYER AG, D-51368 Leverkusen (DE)
(72) Erfinder: Cramm, Günther, Dr., W-5090 Leverkusen 3 (DE); Lindel, Hans, Dr., W-5090 Leverkusen (DE); Steffan, Guido, Dr., W-5068 Odenthal (DE)

(56) Entgegenhaltungen:
- EP-A- 0 178 260
- DE-A- 1 178 052
- DE-A- 1 695 659

## Beschreibung

Es ist bekannt, daß sich 2-Aminopyridine mit Hilfe der Sandmeyer-Reaktion oder ähnlich gelagerter Umsetzungen in die entsprechenden 2-Chlorpyridine überführen lassen. Die besten Ausbeuten werden bei der Umsetzung in einer gesättigten salzsauren, methanolischen Lösung mit Alkylnitriten mit vorzugsweise 3 bis 5 Kohlenstoffatomen erhalten (DE-A 16 95 659). Voraussetzung ist hier ein Molverhältnis 2-Aminopyridin zu Methanol von 1:8 bis 1:12.

Ein wesentlicher Nachteil des Verfahrens nach DE-A 16 95 659 ist darin zu sehen, daß sich die Bildung von Methylchlorid nicht vermeiden läßt, insbesondere bei einer wirtschaftlich vertretbaren Rückgewinnung des Methanols. Ein weiterer Nachteil ist die Verwendung von Alkylnitriten wie Butyl-, Amyl- oder Propylnitrit, weil die daraus entstehenden Alkohole nur aufwendig vom Methanol als Lösungsmittel zu trennen sind.

Es ist weiterhin bekannt, daß man 2-Chlor-pyridine erhält, wenn man Alkyl-2-pyridone in Gegenwart von N,N-disubstituierten Formamiden mit Phosgen umsetzt (z.B. EP-A 72 777) oder 5,6-Dihalogen-2-piperidinone mit POCl₃ oder Phosgen und anschließend mit Basen behandelt (z.B. EP-A 121 320) oder Pyridin-N-oxide mit Chlor enthaltenden Phosphorsäurederivaten umsetzt (z.B. DE-A 3 839 332).

Weiterhin ist bekannt, daß man 2-Chlor-pyridine erhält, wenn man 2-Amino-pyridine in wäßrig-salzsaurer Lösung mit Natriumnitrit umsetzt (z.B. J. Heterocycl. Chem. 2, 1965, 420).

Die Diazotierung in wäßriger Salzsäure liefert nur Ausbeuten von 30 bis max. 50 % an 2-Chlorpyridinen; Hauptprodukte sind die entsprechenden Hydroxylverbindungen.

Es wurde nun gefunden, daß man 2-Chlorpyridin-Derivate der Formel (I) 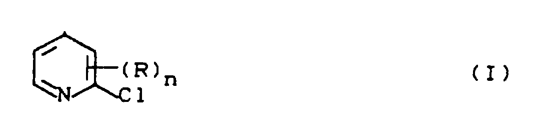 in welcher
- R: für Wasserstoff, C₁-C₄-Alkyl oder Halogen steht und
- n: für 0 bis 4 steht,
in guten Ausbeuten und hoher Reinheit erhält, wenn man ein 2-Aminopyridin-Derivat der Formel (II) 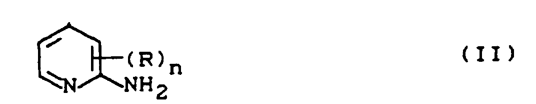 in welcher
- R und n: die oben angegebenen Bedeutungen haben,
in mit Chlorwasserstoff gesättigter wäßriger Lösung bei Temperaturen zwischen -10 und +50°C mit Nitrosylchlorid unter gleichzeitigem Einleiten von Chlorwasserstoff umsetzt.

Die erfindungsgemäße Umsetzung wird vorzugsweise zur Herstellung von Verbindungen der Formel (I) angewandt, in welcher R für Wasserstoff, Chlor oder C₁-C₂-Alkyl steht und n für 1 oder 2 steht. Nach dem erfindungsgemäßen Verfahren wird insbesondere die Verbindung 2-Chlor-5-methyl-pyridin hergestellt.

Überraschenderweise können 2-Chlorpyridin-Derivate nach dem erfindungsgemäßen Verfahren auf relativ einfache Weise in sehr guter Ausbeute und in hoher Reinheit hergestellt werden, während nach den bekannten Verfahren diese Produkte in mäßigen Ausbeuten und unbefriedigender Qualität erhalten werden.

Weitere Vorteile des erfindungsgemäßen Verfahrens liegen in der Verwendung von Wasser als Lösungsmittel (Vorteile bei der Abfall- und Reststoffverwertung bzw. -beseitigung), der völligen Vermeidung der Bildung von Alkylchloriden (wie sie bei Umsetzungen von Alkylnitriten in Methanol auftreten). Weiterhin fallen keine phosphatbelasteten Abwässer an (wie bei Reaktionen mit Phosphorsäure-Derivaten), und es entfällt die sicherheitstechnisch aufwendige Handhabung von Phosgen.

Verwendet man 2-Amino-5-methylpyridin, HCl und Nitrosylchlorid als Ausgangsverbindungen, so kann der Reaktionsablauf wie folgt wiedergegeben werden:

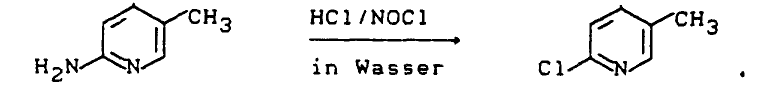

Die als Ausgangsverbindungen zu verwendenden 2-Amino-pyridin-Derivate der Formel (II) sind bekannt. Das zu verwendende Nitrosylchlorid ist ebenfalls bekannt.

Als beim erfindungsgemäßen Verfahren einsetzbare 2-Aminopyridine der Formel (II) seien beispielhaft genannt:

2-Amino-5-methyl-pyridin, 2-Amino-3-methyl-pyridin, 2-Amino-4-methyl-pyridin, 2-Amino-6-methyl-pyridin, 2-Amino-pyridin, 2-Amino-4-chlor-pyridin, 2-Amino-5-ethyl-pyridin, 2-Amino-3,5-dimethyl-pyridin. Besonders bevorzugt wird 2-Amino-5-methyl-pyridin eingesetzt.

Das erfindungsgemäße Verfahren wird durchgeführt, indem man das 2-Aminopyridin-Derivat in HCl-gesättigter wäßriger Lösung mit Nitrosylchlorid umsetzt. Hierbei wird gleichzeitig Chlorwasserstoffgas eingeleitet, so daß die Reaktionsmischung immer mit Chlorwasserstoff gesättigt ist.

Es werden 1 bis 5 Moläquivalente, bevorzugt 1,1 bis 3 Moläquivalente Nitrosylchlorid pro Mol Aminopyridin-Ausgangsverbindung eingesetzt.

Das Verfahren wird im allgemeinen bei Temperaturen zwischen -10°C und +50°C durchgeführt, vorzugsweise zwischen 0°C und +20°C. Es wird im allgemeinen unter Normaldruck gearbeitet.

Nach beendeter Reaktion wird das Reaktionsgemisch eingeengt, dann wird mit Basen (insbesondere mit Alkalihydroxid) alkalisch gestellt und die organische Phase abgetrennt. Aus der organischen Phase wird das 2-Chlorpyridin vorzugsweise durch Destillation in reiner Form erhalten.

Alternativ hierzu kann nach dem Alkalisch-Stellen das Reaktionsprodukt auch durch Azeotrop-Destillation mit Wasser isoliert werden. Das dabei erhaltene Azeotrop kann vom Wasser getrennt und zusammen mit der obengenannten organischen Phase einer Destillation unterworfen werden.

Bei der erfindungsgemäßen Umsetzung entsteht ein Pyridon-2-Derivat als Nebenprodukt, dieses befindet sich beim Aufarbeiten nach dem Abtrennen der organischen Phase in der Mutterlauge, wobei beim weiteren Einengen (Abdestillieren von Wasser) das Pyridon-2-Derivat auskristallisiert. Nach dem Abtrennen des Pyridon-2-Derivats kann dieses in an sich bekannter Weise, z.B. nach dem Verfahren der Deutschen Auslegeschrift Nr. 1 178 052 durch Umsetzung mit Thionylchlorid in Gegenwart eines N,N-dialkyl-substituierten Formamids in das entsprechende 2-Chlorpyridin-Derivat übergeführt werden. Dadurch läßt sich die Gesamtausbeute an 2-Chlor-pyridin der Formel (I) zusätzlich steigern.

Diese Aufarbeitung der Pyridon-2-haltigen Mutterlauge kann als zusätzliche Verfahrensmaßnahme durchgeführt werden.

Das nach dem erfindungsgemäßen Verfahren herstellbare 2-Chlor-5-methyl-pyridin ist als Zwischenprodukt für Pharmazeutika bekannt (vgl. DE-A 28 12 585).

Weiterhin kann 2-Chlor-5-methyl-pyridin als Zwischenprodukt für die Herstellung von insektiziden Nitromethylen-Derivaten eingesetzt werden (vgl. EP-A 163 855).

Weiterhin können die erfindungsgemäß herstellbaren Verbindungen als Zwischenprodukte zur Herstellung von Diazotypiefarbstoffen (vgl. GB-A 870 027) und Haarfarbstoffen (vgl. DE-A 1 142 045) eingesetzt werden.

Schließlich sind die erfindungsgemäß herstellbaren 2-Chlorpyridine wertvolle Zwischenprodukte für die Herstellung von herbizid wirksamen α-[4-(Pyrid-2-yloxy)phenoxy]-alkancarbonsäuren und deren Derivaten (siehe dazu CH-Patent Nr. 622 170).

### Herstellungsbeispiel

Die Lösung von 540 g 2-Amino-5-methyl-pyridin in 500 g Wasser bzw. dem salzsaurem Destillat einer vorausgegangenen Partie (siehe unten) wird bei 0 bis 10°C mit Chlorwasserstoffgas gesättigt. Innerhalb von 6 Stunden wird die Lösung bei 0 bis 5°C simultan mit 400 g Nitrosylchlorid und 300 g Chlorwasserstoff begast. Nach einer weiteren Stunde wird aus der Reaktionslösung die Hauptmenge des freien Chlorwasserstoffs sowie ca. 500 g Wasser abdestilliert. Das Destillat wird beim folgenden Ansatz wieder als Reaktionsmedium eingesetzt.

Der Rückstand wird mit 500 g Wasser bzw. mit beim Einengen (s.u.) erhaltenem Destillat verdünnt und mit 7 Mol Natronlauge bei 70 bis 80°C auf pH 8-9 gestellt. Die sich dabei abscheidende organische Phase wird abgetrennt und die NaCl-gesättigte, wäßrige Phase anschließend auf ein Viertel des Ausgangsvolumen eingeengt.

Dabei geht zunächst noch eine geringe Menge 2-Chlormethylpyridin als Azeotrop über. Es wird vom Wasser getrennt und zusammen mit der organischen Phase über eine Kolonne destilliert. Die Ausbeute an 2-Chlor-5-methylpyridin beträgt 535 g bzw. 83,9 % der Theorie.

Die beim Einengen der wäßrigen Phase erhaltenen ca. 500 g Wasser werden zum Verdünnen des folgenden Ansatzes vor der Neutralisation verwendet. Das nach Abdestillieren von weiteren ca. 250 g Wasser ausgefallene Kochsalz wird heiß abgesaugt. Das beim Abkühlen der Mutterlauge auskristallisierende 5-Methylpyridon-2 wird abgetrennt, die verbleibende Mutterlauge wird dem folgenden Ansatz nach der Phasentrennung zugesetzt und das restliche Pyridon durch Einengen der Mutterlauge, wie zuvor beschrieben, mitisoliert.

Das erhaltene 5-Methylpyridon-2 (87 g bzw. ca. 16 % der Theorie, bezogen auf das eingesetzte Aminomethylpyridin), wird nach bekanntem Verfahren (vgl. Deutsche Auslegeschrift 11 78 052) ebenfalls in 2-Chlor-5-methylpyridin überführt.

Die Gesamtausbeute an 2-Chlor-5-methylpyridin, bezogen auf das eingesetzte Aminomethylpyridin, beträgt 601 g bzw. 94,1 % d.Th.

## Patentansprüche

1. Verfahren zur Herstellung von 2-Chlorpyridin-Derivaten der Formel (I) 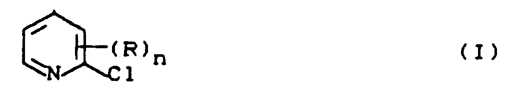 in welcher
R für Wasserstoff, C₁-C₄-Alkyl oder Halogen steht und
n für 0 bis 4 steht,
dadurch gekennzeichnet, daß man 2-Aminopyridin-Derivate der Formel (II) 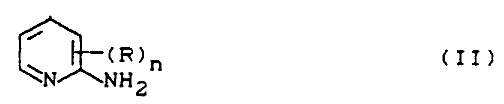 in welcher
R und n die oben angegebenen Bedeutungen haben,
in mit Chlorwasserstoff gesättigter wäßriger Lösung bei Temperaturen zwischen -10 und +50°C mit Nitrosylchlorid unter gleichzeitigem Einleiten von Chlorwasserstoff umsetzt.

2. Verfahren gemäß Anspruch 1, dadurch gekennzeichnet, daß man die Umsetzung bei 0 bis 20°C durchführt.

3. Verfahren gemäß Anspruch 1 bis 2, dadurch gekennzeichnet, daß man das bei der Reaktion als Nebenprodukt entstehende Pyridon nach an sich bekannten Methoden zum 2-Chlor-pyridin-Derivat der Formel (I) aufarbeitet.

4. Verfahren nach Anspruch 1 bis 3, dadurch gekennzeichnet, daß man aus 2-Amino-5-methyl-pyridin das 2-Chlor-5-methyl-pyridin herstellt.

## Claims

1. Process for the preparation of 2-chloropyridine derivatives of the formula (I) 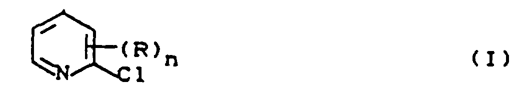 in which
R represents hydrogen, C₁-C₄-alkyl or halogen and
n represents 0 to 4,
characterized in that 2-aminopyridine derivatives of the formula (II) 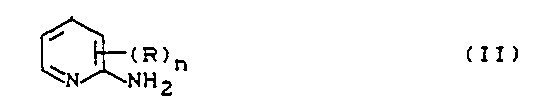 in which
R and n have the above definitions,
are reacted with nitrosyl chloride in a hydrogen chloride-saturated aqueous solution at temperatures between -10 and +50°C with simultaneous introduction of hydrogen chloride.

2. Process according to Claim 1, characterized in that the reaction is carried out at 0 to 20°C.

3. Process according to Claims 1 and 2, characterized in that the pyridone resulting as a by-product in the reaction is worked up to give the 2-chloropyridine derivative of the formula (I) by methods known per se.

4. Process according to Claims 1 to 3, characterized in that 2-chloro-5-methyl-pyridine is prepared from 2-amino-5-methyl-pyridine.

## Revendications

1. Procédé pour la préparation de dérivés de 2-chloropyridines de formule (I) 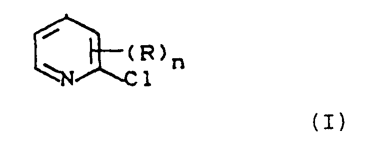 dans laquelle
R représente un atome d'hydrogène, un groupe alkyle en C₁-C₄ ou un atome d'halogène et
n représente de 0 à 4,
caractérisé en ce qu'on fait réagir des dérivés de 2-aminopyridines de formule (II) 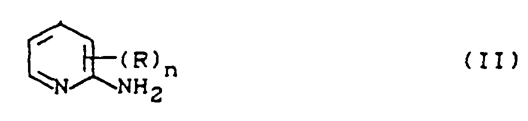 dans laquelle
R et n ont les significations indiquées ci-dessus,
dans une solution aqueuse saturée avec de l'acide chlorhydrique, à des températures entre -10 et +50°C, avec du chlorure de nitrosyle, tout en introduisant simultanément de l'acide chlorhydrique.

2. Procédé selon la revendication 1, caractérisé en ce qu'on effectue la mise en réaction à une température de 0 à 20°C.

3. Procédé selon les revendications 1 à 2, caractérisé en ce qu'on traite la pyridone obtenue sous forme de sous-produit lors de la réaction, conformément à des procédés connus en soi pour obtenir le dérivé de 2-chloropyridine de formule (I).

4. Procédé selon les revendications 1 à 3, caractérisé en ce qu'on prépare la 2-chloro-5-méthylpyridine à partir de la 2-amino-5-méthylpyridine.
